(19) Europäisches Patentamt
European Patent Office
Office européen des brevets

(11) Publication number: 0 018 632
B1

# (12) EUROPEAN PATENT SPECIFICATION

(45) Date of publication of patent specification: 15.06.83

(51) Int. Cl.³: C 08 L 77/00, C 08 K 5/06, C 08 K 5/36, C 08 K 3/20

(21) Application number: 80102340.9

(22) Date of filing: 30.04.80

## (54) Thermally stable, flame-retardant thermoplastic polyamide resins.

(30) Priority: 01.05.79 US 35097

(43) Date of publication of application:
12.11.80 Bulletin 80/23

(45) Publication of the grant of the patent:
15.06.83 Bulletin 83/24

(84) Designated Contracting States:
BE CH DE FR GB IT LI NL SE

(56) References cited:
FR - A - 2 010 401
FR - A - 2 044 638
FR - A - 2 113 965
FR - A - 2 225 477
GB - A - 1 160 716
US - A - 3 760 003

PATENTS ABSTRACTS OF JAPAN, vol. 1, no. 10, 18th March 1977, page 347C76

(73) Proprietor: E.I. DU PONT DE NEMOURS AND COMPANY
Legal Department 1007 Market Street
Wilmington Delaware 19898 (US)

(72) Inventor: Jones, Richard Hamilton
233 Cheltenham Road
Newark, Delaware 19711 (US)

(74) Representative: Abitz, Walter, Dr.-Ing. et al,
Abitz, Morf, Gritschneder P.O. Box 86 01 09
D-8000 München 86 (DE)

EP 0 018 632 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Thermally stable, flame-retardant thermoplastic polyamide resins

## Field of the Invention

This invention relates to thermally stable, flame-retardant thermoplastic polyamide resins containing oligomers of aromatic diols and brominated diphenyl ethers.

## Background of the Invention

Heretofore, brominated compounds have been employed in thermoplastic polymers to make resins which have improved flame-retardance over the thermoplastic polymer alone (see e.g. US - A - 3,760,003; FR - A - 2,044,638, FR - A - 2,113,965 and 2,225,477). However, many such brominated compounds, such as decabromodiphenyl ether (FR - A - 2,225,477), tend to degrade these polymers at melt-processing temperatures, and to migrate in articles molded from the resin toward the surface of the molded article.

It has now been discovered that certain brominated oligomers which when employed with thermoplastic polymers result in flame retardant resins which have better stability toward thermal degradation than do resins which contain some known brominated flame retardants. These oligomers do not exhibit any substantial tendency to migrate toward the surface of articles molded from the resins.

## Summary of the Invention

The oligomers are represented by the formula

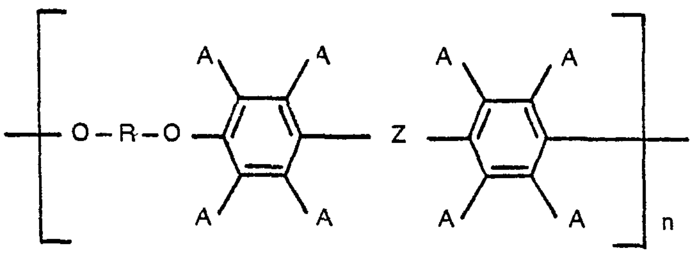

wherein R is a divalent arylene group of between about 6—15 carbon atoms, and preferably is

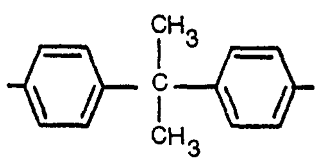 or 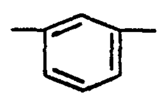

Z is 0, S,

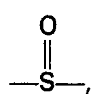, or 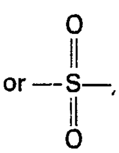, each A is

independently hydrogen or bromine with the proviso that at least six of the A groups are bromine; and n is a cardinal number of between 2 and 20.

The flame retardant polyamide resins of the invention consist of

a) a flame-retardant thermoplastic polyamide resin,

b) between 1—30% by weight, based on the total weight of the composition, of an oligomer represented by the formula

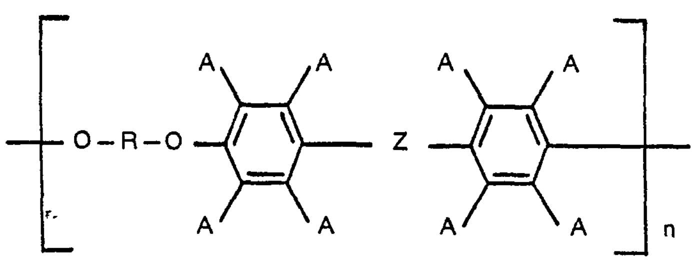

wherein R is a divalent arylene group of between 6—15 carbon atoms; each A is independently hydrogen or bromine with the proviso that at least six of tne A groups are bromine; n is a cardinal number of between 2 and 20; and

Z is 0, S, 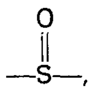, or 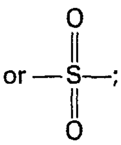;

c) and between 1—15 percent by weight, based on the total weight of the composition, of a metal oxide synergist that is compatible with the polymer. The metal oxide synergist enhances flame retardancy of the polyamide resin.

## Description of the Invention

The oligomers are prepared by reacting an aromatic diol of the formula HO—R—OH wherein R is as previously defined with a brominated compound of the formula

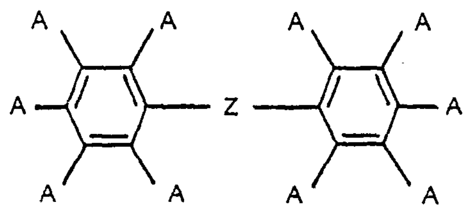

wherein each A and Z are as defined above.

The aromatic diols employed in preparing the oligomers are diols containing at least one aromatic nucleus, i.e., the R group in the formula HO—R—OH is an arylene group, i.e., the —OH groups are attached directly to an aryl nucleus. For example, the group may be, phenylene, biphenylene, naphthalene or two aromatic benzene rings separated by an alkylidene group e.g.,

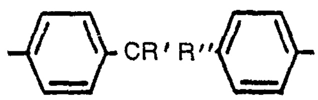

where each R′ or R″ can separately be hydrogen or lower alkyl (1—6 carbon atoms).

Bridging groups, such as defined by Z in the formula of the oligomers, may be used to connect the two aromatic rings. Representative diols include resorcinol, "Bisphenol A" (2,2-bis-(p-hydroxy phenyl)propane), hydroquinone, 4,4′-biphenol, 4,4′-dihydroxybenzophenone and 4,4′-dihydroxydiphenyl sulfone. Resorcinol and "Bisphenol A" are preferred.

Representative brominated compounds employed preparing the oligomers include decabromodiphenyl ether, decabromodiphenyl sulfide, and octabromodiphenyl ether.

The process for preparing the oligomers is preferably carried out in a solvent for at least one and preferably both the monomers. Suitable solvents include dimethyl acetamide, dimethyl formamide, dimethyl sulfoxide, N-methyl pyrrolidone, tetramethylene sulfone, and hexamethylphosphoramide, which may be used alone or in combination with an aromatic solvent such as benzene, toluene, xylene, chlorobenzene, or o-dichlorobenzene.

The reaction mixture is maintained at a temperature between 25° and 250°C. For convenience, reflux temperature can be employed. Pressure is not critical and super- or sub-atmospheric pressure can be used, but conveniently atmospheric pressure is employed. The oligomers are usually prepared by reacting substantially equimolar portions of the aromatic diol and brominated compound, although slight excesses of either component can be used to limit molecular weight. Generally, the brominated compound and the aromatic diol will be present in the solvent or solvent mixture in amounts of between 10 and 300 grams per liter of solvent. Time of reaction is not critical but generally ranges from 1—8 hours.

Suitable bases used to carry out the reaction include alkali metal, alkaline earth, or quaternary ammonium hydroxides or salts of weak acids, such as potassium carbonate, sodium carbonate, magnesium oxide, sodium hydroxide, potassium hydroxide and calcium hydroxide. Besides *in situ* contacting of the aromatic diol with the base, a pre-prepared salt of the formula M—O—R—O—M where M is an alkali metal, alkaline earth metal, or quaternary ammonium ion and R has the meaning defined above, may be used directly.

If desired, the oligomer may be capped with phenol or a similar compound to improve thermal stability. Alternatively, the reaction can be terminated with methyl chloride or ethyl bromide or the like

to provide stable alkyl ends on the oligomer. At the end of the reaction, the oligomer is precipitated by adding the reaction mixture to a non-solvent for the oligomer, such as methanol. The precipitate is then collected and dried.

To prepare the flame retardant resins of this invention, the oligomer and the thermoplastic polyamide are blended by mixing them by any convenient means. As previously mentioned, the oligomer is added in amounts of between 1 and 30 percent based on total weight, and preferably between 10 and 25 percent.

A metal oxide enhances the flame-retardant nature of the resins and the resins of this invention contain between about 1—15 percent, preferably 3—10 percent, by weight based on total weight, of a metal oxide. Preferred metal oxides include antimony oxide, iron oxide, zinc oxide and zinc ferrite. The particular metal oxide selected is one that is compatible with the thermoplastic polyamide, i.e., it does not tend to degrade the polyamide employed.

Thermoplastic polyamides useful in this invention include polyhexamethylene adipate or polycaprolactam. The polyamides are high polyamides of film-forming molecular weight. The polyamides can be copolyamides such as nylon 66/6 or can be physical blends of polyamides.

The polyamides are well known in the art and are of film-forming molecular weight. The polyamide resin can be produced by condensation of equimolar amounts of a saturated organic dicarboxylic acid containing from 4—12 carbon atoms with an organic diamine containing 2—13 carbon atoms, in which the diamine can be employed, if desired, to provide an excess of amine end groups over carboxyl end groups in the polyamide or, vice versa, an excess of diacid can be used. Equally well, these polyamides may be made from amine-forming and acid-forming derivatives of said amines and acids such as esters, acid chlorides, amine salts, etc. Representative dicarboxylic acids used to make the polyamides include adipic acid, pimelic acid, suberic acid, sebacic acid, and dodecanedioic acid, while representative diamines include hexamethylenediamine and octamethylenediamine. In addition, the polyamide may be prepared from self-condensation of a lactam. Examples of polyamides include polyhexamethylene adipamide (66 nylon), polyhexamethylene azelaamide (69 nylon), polyhexamethylene sebacamide (610 nylon), and polyhexamethylene dodecanoamide (612 nylon), polybis-(4-aminocyclohexyl)methane dodecanoamide, or the polyamides produced by ring opening of lactams, i.e., polycaprolatam, (6 nylon), polylauryl lactam, or poly-11-aminoundecanoamide. It is also possible to use polyamides prepared by the co-polymerization of two of the above polymers or terpolymerization of the above polymers or their components, as for example, a polymer made of adipic acid, and isophthalic acid and hexamethylene diamine. Blends of polyamides, such as a mixture of 66 nylon and 6 nylon are also included. Preferably the condensation polyamide employed herein is polyhexamethylene adipamide (66 nylon).

Fillers and reinforcing agents such as glass fibers, graphite fibers, mica, wollastonite and aluminum silicate, may be used in the polymer composition to modify properties such as stiffness and toughness. Pigments can also be used.

In the Examples which follow thermal stability of the resins obtained therein was determined by measuring the melt index (a measurement that provides a number which is a function of the molecular weight of the thermoplastic polymer in the resin — the higher the melt index the lower the molecular weight of the polymer measured) in a standard melt index apparatus as a function of time using ASTM method D1238—73. The apparatus had a 2.09 mm orifice and a 2170 g. weight was used to force the polymer through the orifice at a temperature of 280°C or 300°C.

Tensile strength and elongation measurements were measured as described in ASTM D638—77a except that 3 specimens rather than 5 were tested and samples were not conditioned at 50% relative humidity but, rather, were tested dry as molded (DAM) after conditioning for 24 hours under nitrogen at 23°C.

Flexual modulus was measured as described in ASTM D790—71 except that 3 rather than 5 specimens were tested and conditioning was carried out for 24 hours under nitrogen at 23°C (DAM).

Notched Izod values were determined by the procedure described in ASTM D256—73. Samples were tested dry as molded (DAM) after conditioning for 24 hours under nitrogen at 23°C.

Example 1

A. *Preparation of oligomer*

An oligomer was prepared by heating 33.0 grams (0.30 mole) resorcinol with 300 grams (0.313 mole) decabromodiphenyl ether (DBDPE) in 600 ml chlorobenzene and 600 ml dimethyl acetamide (DMAC). Twenty-seven grams of sodium hydroxide flake was added as the base required to carry out the reaction. The mixture was heated at reflux (146°C) for 3 hours and water formed by the reaction was removed using a modified Dean Stark trap. The reaction mixture was filtered hot and the oligomer was precipitated by pouring the reaction mixture into 3 liters of methanol. The solid product was collected and dried. Oligomer recovered was 194 grams. The oligomer had an inherent viscosity of 0.032 dl/g when measured at 0.5 g/100 ml. chlorobenzene at 30°C. The number average molecular weight of the product was 2770 as measured by vapor pressure osmometry in o-dichlorobenzene at 100°C.

B. *Preparation of polyamide composition*

1. A flame-retardant polyamide resin (66 nylon) composition was prepared by mixing 5% zinc ferrite and 12% of the oligomer prepared in part A with a 90%/10% 66/6 polyamide copolymer having an intrinsic viscosity above 1.0 in a Brabender mixer for 5 minutes at 260°C. Samples of the resin were molded into 1.6 mm bars at 270°C and 11 MPa pressure, and tested for flammability by Underwriter's Laboratory (UL) Test 94. The samples were found to have a flammability rating of V—O by this test and an average burn time of 3.8 seconds.

2. Melt index values of an additional composition of the oligomer in 66 nylon having an intrinsic viscosity above 1.0 were obtained at 5 minute intervals so that the progressive degradation of polymer at 280°C could be obtained. As shown by the data below, the flame-retardant polyamide resin was more thermally stable than a similar polyamide resin containing decabromodiphenyl ether (DBDPE), a known flame retardant, in place of the oligomer.

| Composition | | |
|---|---|---|
| Bromine Compound | DBDPE | Resorcinol-DBDPE Oligomer |
| Amount of Bromine Compound | 2.0 g | 2.4 g |
| Wgt. % Bromine in Composition | 8.3% | 8.4% |
| $ZnFe_2O_4$ | 1.0 g | 1.0 g |
| 66 Nylon | 17.0 g | 16.4 g |
| **Melt Index (g/min. at 280°C)** | | |
| After 5 mins. | 4.62 | 3.23 |
| After 10 mins. | 13.8 | 2.04 |
| After 15 mins. | | 2.12 |

Example 2

A. *Preparation of oligomer*

750 grams of decarbromodiphenyl ether (0.78 mole) and 178.5 grams (0.78 mole) of 2,2-bis-(p-hydroxyphenyl) propane were placed in a four liter resin kettle along with 1.5 l. dimethyl acetamide and 1.5 l. chlorobenzene. The mixture was stirred and heated under nitrogen to 130°C. Then, 3.3 g. of sodium hydroxide was added and the mixture was refluxed for one hour at 146°C. Then, an additional 33.3 g. of sodium hydroxide was added. The mixture was refluxed at 146°C for a total of four hours while the water of reaction was continually removed with a modified Dean Stark trap. At the end of this time the solution was filtered hot to remove sodium bromide and the oligomer was precipitated by pouring the filtered reaction solution into 4 l. of rapidly stirred methanol. The precipitate was thoroughly washed with methanol, collected by filtration, and dried. The weight of dried product was 688 grams. The inherent viscosity of the product was 0.04 dl/g when measured at 0.5 g/100 ml chlorobenzene at 30°C. The number average molecular weight was determined to be 4110, by vapor pressure osmometry in o-dichlorobenzene at 100°C.

B. *Preparation of polyamide composition*

The oligomer was compounded with 66 nylon having an intrinsic viscosity over 1.0 at 288°C melt temperature in a 28 mm twin screw extruder to give a composition containing 5% (wgt.) zinc ferrite and 15% (wgt.) of the oligomer. The resulting composition was extruded into strands and chopped into pellets. The pellets were used to measure melt index at 300°C and were injection molded to give test bars for flammability testing by UL 94. For comparison purposes 66 nylon was compounded with 5% (wgt.) zinc ferrite and 12% decabromodiphenyl ether and injection molded in a similar manner. Results obtained with the two compositions are shown in the data below which demonstrates that the oligomers prepared in this example gave a much more thermally stable composition than decabromodiphenyl ether.

| Bromine Source | 15% Example 2 Oligomer | 12% Decabromodiphenyl Ester |
|---|---|---|
| Bromine Content (wgt.%) | 9.3% | 10.0% |
| UL 94 Test | | |
| Burn Time (seconds) | 3.8 | 2.8 |
| Rating | V—O | V—O |
| Melt Index at 300°C (g./min.) | | |
| After 5 mins. | 2.60 | 83 |
| After 10 mins. | 3.57 | — |
| After 15 mins. | 6.75 | — |

Example 3

A. *Preparation of oligomer*

An oligomer was prepared by heating 68.4 grams (0.30 moles) of "Bisphenol A" with 165.6 grams (0.30 moles) hexabromobenzene in 400 ml. N,N-dimethylacetamide and 140 ml. of monochlorobenzene. Anhydrous potassium carbonate (83 grams) was added as the base required to carry out the reaction. The mixture was heated under nitrogen at 151° for three hours while removing water with a modified Dean Stark trap. Solids were filtered off and the oligomer was end-capped with 30 ml. of ethyl bromide. The oligomer was precipitated in methanol, washed with methanol and dried in a vacuum oven.

B. *Preparation of polyamide composition*

1. A mixture of 4.8 g. antimony trioxide, 12.0 g. of the "Bisphenol A" / decabromodiphenyl ether oligomer prepared above of inherent viscosity 0.02 dl/g at 0.5 g/100 ml. chlorobenzene at 30°, and 43.2 g. of a 66 nylon having an intrinsic viscosity above 1.0 and a number average molecular weight of about 18,000 were compounded in a Brabender mixer for 10 minutes at 270°C. The sample was compression molded at 275°C into a 1.6 mm x 12.7 mm x 127 mm bar and tested for flammability by the procedure of UL 94, except 3 rather than 5 bars were tested. The sample was rated V—O and had an average burn time of 0.2 seconds.

2. When the mixture of part 1 was used except that 30.2 g. of the 66 nylon were used and 13.0 g. of 1.6 mm glass fibers were added, and was compression molded at 275°C into 1.6 mm x 12.7 mm x 127 mm bars and tested for flammability by UL 94, using 3 bars rather than 5 bars as specified, the sample was rated V—O and had an average burn time of 0.0 seconds.

Intrinsic viscosity of the nylons used herein was determined in 90% formic acid at 25°C.

**Claims**

1. A flame-retardant resin consisting of

a) a flame-retardant thermoplastic polyamide resin,

b) between 1—30% by weight, based on the total weight of the composition, of an oligomer represented by the formula

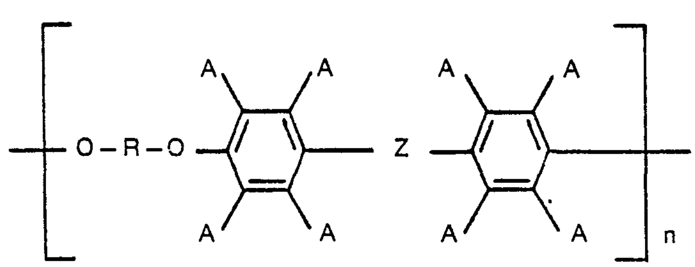

wherein R is a divalent arylene group of between 6—15 carbon atoms; each A is independently hydrogen or bromine with the proviso that at least six of the A groups are bromine; n is a cardinal number of between 2 and 20; and

Z is O, S, 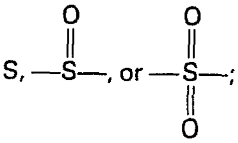 , or ;

c) and between 1—15 percent by weight, based on the total weight of the composition, of a metal oxide synergist that is compatible with the polymer.

2. The resin of Claim 1 wherein the polyamide resin is polyhexamethylene adipamide.

3. The resin of Claim 1 wherein the polyamide resin is a mixture of polyhexamethylene adipamide and polycaprolactam.

4. The resin of Claims 1 to 3 which contains a filler or reinforcing agent.

5. The resin of Claims 1 to 4 which contains glass fibers.

**Revendications**

1. Résine à combustion retardée comprenant:

a) une résine polyamide thermoplastique à combustion retardée,

b) entre 1 et 30% en poids, par rapport au poids total de la composition, d'un oligomère représenté par la formule:

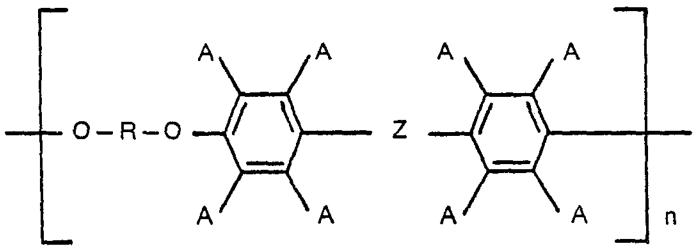

dans laquelle R est un groupe arylène divalent de 6 à 15 atomes de carbone, chaque A est indépendamment un atome d'hydrogène ou de brome avec la condition qu'au moins six des groupes A sont des atomes de brome, *n* est un numbre cardinal de 2 à 20, et

Z est O, S, 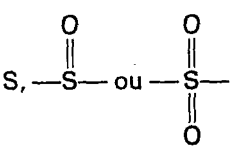 ou

c) et entre 1 et 15% en poids, par rapport au poids total de la composition, d'un agent synergique du type oxyde de métal qui est compatible avec le polymère.

2. Résine selon la revendication 1, dans laquelle la résine polyamide est du poly-hexaméthylène-adipamide.

3. Résine selon la revendication 1, dans laquelle la résine polyamide est un mélange de poly-hexaméthylène adipamide et de polycaprolactame.

4. Résine selon les revendications 1 à 3, qui contient une charge ou un agent de renforcement.

5. Résine selon les revendications 1 à 4, qui contient des fibres de verre.

**Patentansprüche**

1. Flammhemmendes Harz, bestehend aus

a) einem flammhemmenden thermoplastischen Polyamidharz,

b) zwischen 1—30 Gew.-%, basierend auf dem Gesamtgewicht der Zusammensetzung, eines Oligomeren, dargestellt durch die Formel

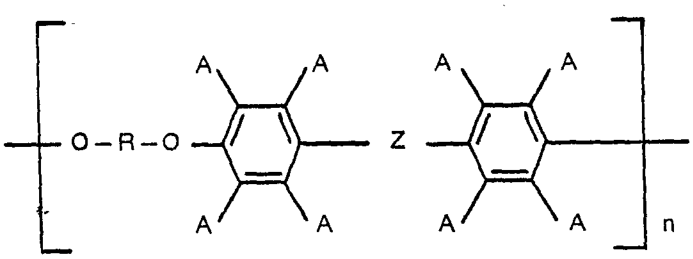

worin R eine zweiwertige Arylengruppe mit zwischen 6—15 Kohlenstoffatomen ist; jedes A unabhängig Wasserstoff oder Brom ist, mit der Maßgabe, daß mindestens sechs der Gruppen A Brom sind; n eine Grundzahl von zwischen 2 und 20 ist; und

Z O, S, 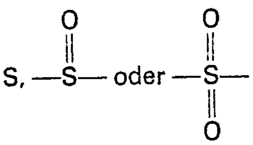 oder ist;

c) und zwischen 1—15 Gew.-%, basierend auf dem Gesamtgewicht der Zusammensetzung, eines Metalloxid-Synergisten, der mit dem Polymeren verträglich ist.

2. Harz nach Anspruch 1, in dem das Polyamidharz Polyhexamethylenadipamid ist.

3. Harz nach Anspruch 1, in dem das Polyamidharz ein Gemisch von Polyhexamethylenadipamid und Polycaprolactam ist.

4. Harz nach den Ansprüchen 1 bis 3, das einen Füllstoff oder ein Verstärkungsmittel enthält.

5. Harz nach den Ansprüchen 1 bis 4, das Glasfasern enthält.